(19) Europäisches Patentamt
European Patent Office
Office européen des brevets

(11) Publication number: **0 002 859**
**B1**

(12) **EUROPEAN PATENT SPECIFICATION**

(45) Date of publication of patent specification: **11.01.84**

(51) Int. Cl.³: **C 07 D 209/52,**
A 01 N 43/38

(21) Application number: **78200365.1**

(22) Date of filing: **13.12.78**

(54) **3,4-Methano pyrrolidine derivatives, compositions thereof and use as plant-growth regulating agents.**

(30) Priority: **22.12.77 GB 5350277**

(43) Date of publication of application:
**11.07.79 Bulletin 79/14**

(45) Publication of the grant of the patent:
**11.01.84 Bulletin 84/2**

(84) Designated Contracting States:
**BE DE FR GB IT LU NL SE**

(56) References cited:
**CHEMICAL ABSTRACTS, vol. 77, no. 19, November 6, 1972. COLUMBUS, OHIO, USA NORRIS, R.D et al. "Substrate discrimination by prolyl-t RNA synthetase from various higher plants", page 130, abstract 123278u**

**CHEMICAL ABSTRACTS, vol. 70, no. 16, April 21, 1969. Columbus, Ohio, USA FOWDEN L. et al. "Cyclopropane amino acids from Aesculus and Blighia", page 81, abstract 75105a**

**The file contains technical information submitted after the application was filed and not included in this specification**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Devlin, Barry Roy John**
**59 Ruins Barn Road**
**Sittingbourne Kent (GB)**
Inventor: **Kerr, Michael William**
**"Long John", Silver Street**
**Bredgar nr. Sittingbourne Kent (GB)**

(74) Representative: **Hunter, Keith Roger Ian et al,**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 002 859 B1

3,4-Methano pyrrolidine derivatives, compositions thereof and use as plant-growth regulating agents

The present invention relates to novel 3,4-methano pyrrolidine derivatives which exhibit interesting biological activity. The present invention also relates to compositions comprising as active ingredient a 3,4-methano pyrrolidine derivative according to the invention. The present invention further relates to a method for sterilizing male anthers in plants, in particular in small-grain cereals, by applying thereto a compound according to the present invention or a composition comprising at least a compound according to the present invention. The compounds exhibit useful herbicidal activity.

In our Belgium Patent No. 846,125 it has been disclosed that certain 3,4-methano pyrrolidine derivatives, especially 2-carboxy-3,4-methano pyrrolidine and 2-methoxycarbonyl-3,4-methano pyrrolidine exhibit useful activity as sterilants for male anthers in plants. The compounds of this Belgium patent are not shown to be herbicidal, in fact, according to the reference teaching they can be usefully applied to plants at higher dosage rates to as plant growth regulators without any killing or herbicidal effects.

Fowden and Smith in "Phytochemistry" 1969, vol. 8, pp. 437, disclose the isolation of cis-L-2-carboxy-3,4-methano-pyrrolidine and its probable biogenetic precursor, cis-$\alpha$(carboxycyclo-propyl)glycine from *A. parviflora* seed. According to this article, the probable biogenetic precursor is a potent inhibitor of mung bean growth. Cis(exo)-3,4-methanopyrrolidine and related proline esters and amides are disclosed as being effective substrates with Prolyl-L-RNA synthetase in an article by Norris and Fowden in 'Phytochemistry' 1972, vol. 11, pp. 2921 to 2935.

It has now been found that compounds of the general formula:

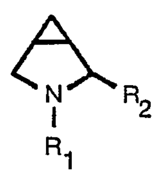

(I)

or the hydrogen halide acid addition salts thereof, wherein $R_1$ represents a hydrogen atom, an alkyl group, or a group of formula

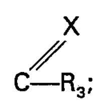

$R_2$ represents $CH_2OH$, a group of formula

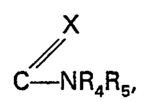

a group of formula

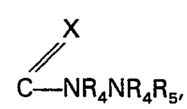

or a group of formula

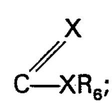

$R_3$ represents a hydrogen atom, an alkyl, alkoxy or alkylthio group, or a group of formula —$NR_4R_5$; each $R_4$ and $R_5$ independently represents a hydrogen atom or an alkyl group; $R_6$ represents a hydrogen atom, an alkyl group, a metal ion of (sub)group I or II of the Periodic Table, an ammonium ion or an alkyl-substituted ammonium ion; and each X independently represents oxygen or sulphur; with the proviso that if $R_1$ represents a hydrogen atom, $R_2$ does not represent a group of formula $CO_2R_6$, exhibit useful biological properties.

The invention also provides as novel compounds, hydrogen halide acid addition salts of compounds of the general formula I given above, in which $R_1$ represents a hydrogen atom and $R_2$ represents a group of formula $CO_2R_6$ where $R_6$ represents a hydrogen atom or an alkyl group.

Preferred compounds according to the invention include those of formula I in which $R_1$ represents a hydrogen atom, an alkyl group having up to 6 carbon atoms, or a group of formula

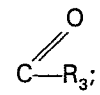

$R_2$ represents a group $CH_2OH$,

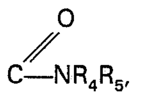 , 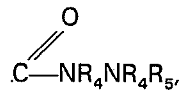 , or 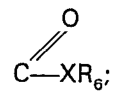 ;

$R_3$ represents a hydrogen atom, an alkyl or alkoxy group having up to 6 carbon atoms, or a group $NR_4R_5$; and each of $R_4$ and $R_5$ independently represent a hydrogen atom or an alkyl group having up to 6 carbon atoms.

Most preferably, $R_1$ represents a hydrogen atom or a methyl group.

It will be appreciated that the pyrrolidine derivatives according to the present invention may exhibit geometrical and/or optical isomerism. The individual isomers as well as mixtures thereof are within the scope of the present invention. It will also be appreciated that certain isomers of the pyrrolidine derivatives are likely to have more activity than the others and that it may be desirable to use the more active component which may have to be separated from the corresponding racemate or may be produced by a stereospecific process.

The pyrrolidine derivatives according to the present invention may be prepared by various methods including those which are analogous to methods known in the art. A preferred method for the preparation of the compounds of formula (I) consists in that a 2-carboxy-3,4-methanopyrrolidine is used as starting material and, in one or more reaction steps is converted to a compound of formula:

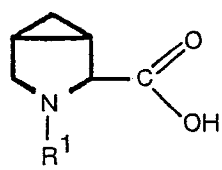

(II)

wherein $R_1$ has the meaning as defined hereinbefore with omission of a hydrogen atom or to a compound of formula:

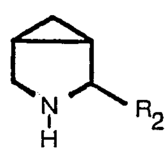

(III)

wherein $R_2$ has the meaning as defined hereinbefore and that optionally the compound of formula (II) or the compound of formula (III) is further converted into the desired compound of formula (I). For example, the alkyl esters of 2-carboxy-3,4-methanopyrrolidine can be suitably prepared by reacting the corresponding acid with the appropriate alkanol in the presence of an acidic catalyst, such as hydrogen chloride. The corresponding amides can be suitably prepared by reacting the appropriate ester (or its acid salt) with an amine. The N-formyl derivative can be prepared by reacting 2-methoxycarbonyl-3,4-methano pyrrolidine with formyl acetic anhydride in a suitable solvent, such as benzene.

The pyrrolidine derivatives may be applied to the plants at a number of stages in their development, suitably during the period between the 2-node stage of the plant and just before emergence of the ear, i.e., during the stem extension period of the plant. When applied during this period substantial sterilization of the male anthers occurs.

The compounds are preferably applied to small-grain cereals, such as wheat, barley, oat, rye, rice and millet, but can also be applied to large-grain cereals, such as maize and sorghum.

The compounds may be applied to the plants as such but, as is normal practice in the application of chemicals to plants, it is preferred to apply the compound in the form of a composition which in addition to the compound also comprises a carrier or a surface-active agent or both a carrier and a surface-active agent.

It has also been found that pyrrolidine derivatives according to the present invention exhibit herbicidal properties, especially post-emergence activity against soya bean. The present invention therefore also relates to a method of controlling or eradicating weeds which comprises applying at a locus a herbicidally active amount of a pyrrolidine derivative according to the present invention or a composition comprising as active ingredient a pyrrolidine derivative according to the present invention. Some interesting herbicidal activity has been found using the pyrrolidine heptyl ester hydrochloric acid salt.

The dosage of the pyrrolidine derivatives applied to the plants to cause male sterility may vary over a wide range but effective results have been obtained for cereal plants by using dosages in the range of from 0.05 kg/ha to 2.00 kg/ha. Compositions containing 50 to 1,500 ppm of pyrrolidine compound are very suitable for this purpose. Weed control can be achieved by applications in the range 0.05 to 10 kg/ha of the compositions comprising as active ingredient a pyrrolidine derivative according to the present invention.

The term "carrier" as used herein means a material, which may be inorganic or organic and of synthetic or natural origin, with which the active compound is mixed or formulated to facilitate its application to the plant, seed, soil or other object to be treated, or its storage, transport or handling. It does not include a surface-active agent as hereinafter described. The carrier may be a solid or a fluid. Any of the materials usually employed in formulating herbicides may be used as the carrier.

Suitable solid carriers are natural and synthetic clays and silicates, for example natural silicas, such as diatomaceous earths; magnesium silicates, for example, talcs; magnesium aluminium silicates, for example, attapulgites and vermiculites; aluminium silicates, for example, kaolinites, montmorillinites and micas; calcium carbonates; calcium sulphate; synthetic hydrated silicon oxides and synthetic calcium or aluminium silicates; elements, such as for example, carbon and sulphur; natural and synthetic resins, such as for example, coumarone resins, polyvinyl chloride and styrene polymers and copolymers; solid polychlorophenols; bitumen; waxes, such as for example, beeswax, paraffin wax, and chlorinated mineral waxes; and solid fertilizers, for example, superphosphates.

Examples of suitable fluid carriers are water, alcohols, such as for example, isopropanol, glycols; ketones, such as for example, acetone, methyl ethyl ketone, methyl isobutyl ketone and cyclohexanone; ethers; aromatic hydrocarbons, such as for example, benzene, toluene and xylene; petroleum fractions, such as for example, kerosine, light mineral oils; chlorinated hydrocarbons, such as for example, carbon tetrachloride, perchloroethylene, trichloroethane, including liquefied normally vaporous gaseous compounds. Mixtures of different liquids are often suitable.

The surface-active agent may be an emulsifying agent or a dispersing agent or a wetting agent; it may be non-ionic or ionic. Any of the surface-active agents usually applied in formulating herbicides or insecticides may be used. Examples of suitable surface-active agents are the sodium or calcium salts of polyacrylic acids and lignin suphonic acids; the condensation products of fatty acids or aliphatic amines or amides containing at least 12 carbon atoms in the molecule with ethylene oxide and/or propylene oxide; fatty acid esters of glycerol, sorbitan, sucrose of pentaerythritol; condensates of these with ethylene oxide and/or propylene oxide; condensation products of fatty alcohols or alkyl phenols, for example, *p*-octylphenol or *p*-octylcresol, with ethylene oxide and/or propylene oxide, sulphates or sulphonates of these condensation products; alkali or alkaline earth metal salts, preferably sodium salts, of sulphuric or sulphonic acid esters containing at least 10 carbon atoms in the molecule, for example, sodium lauryl sulphate, sodium secondary alkyl sulphates, sodium salts of sulphonated castor oil, and sodium alkylaryl sulphonates, such as sodium dodecyl benzene sulphonate; and polymers of ethylene oxide and copolymers of ethylene oxide and propylene oxide.

The compositions of the invention may be formulated as wettable powders, dusts, granules, solutions, emulsifiable concentrates, emulsions, suspension concentrates and aerosols. Wettable powders are usually compounded to contain 25, 50 or 75%w of toxicant and usually contain, in addition to solid carrier, 3—10%w of a dispersing agent and, where necessary, 0—10%w of stabilizer(s) and/or other additives, such as penetrants or stickers. Dusts are usually formulated as a dust concentrate having a similar composition to that of a wettable powder but without a dispersant, and are diluted in the field with further solid carrier to give a composition usually containing 0.5—10%w of toxicant. Granules are usually prepared to have a size between 10 and 100 BS mesh, and may be manufactured by agglomeration or impregnation techniques. Generaly, granules will contain 0.5—25%w of toxicant and 0—10%w of additives, such as stabilizers, slow-release modifiers and binding agents. Emulsifiable concentrates usually contain, in addition to the solvent and, when necessary, co-solvent, 10—50% w/v of toxicant, 2—20% w/v of emulsifiers and 0—20% w/v of appropriate additives, such as stabilizers, penetrants and corrosion inhibitors. Suspension concentrates are compounded so as to obtain a stable, non-sedimenting, flowable product and usually contain 10—75%w of toxicant, 0.5—15%w of dispersing agents, 0.1—10%w of suspending agents, such as protective colloids and thixotropic agents, 0—10%w of appropriate additives, such as defoamers, corrosion inhibitors, stabilizers, penetrants and stickers, and as carrier, water or an organic liquid in which the toxicant is substantially insoluble; certain organic solids or inorganic salts may be dissolved in the carrier to assist in preventing sedimentation or as anti-freeze agents for water.

The compositions of the invention may contain other ingredients, for example, protective colloids, such as gelatin, glue, casein, gums, cellulose ethers, and polyvinyl alcohol; thixotropic agents, e.g., bentonites, sodium polyphosphates; stabilizers, such as ethylene diamine tetra-acetic acid, urea, triphenyl phosphate; other herbicides or pesticides; and stickers, for example, non-volatile oils.

Aqueous dispersions and emulsions, for example, compositions obtained by diluting a wettable powder or an emulsifiable concentrate according to the invention with water, also lie within the scope of the present invention.

The said emulsions may be of the water-in-oil or of the oil-in-water type, and may have a thick "mayonnaise"-like consistency.

The invention is further illustrated in the following Examples. The terminology used to describe the growth stages in the development of cereal plants is that as given in "Plant Pathology", Volume 3, 1954.

Example 1

Preparation of cis-L-N-methyl-3,4-methano-pyrrolidine amide

Cis-L-2-ethoxycarbonyl-3,4-methano-pyrrolidine, hydrochloric acid salt (3.0 g) in ethanol (20 $cm^3$) was heated with a large excess of ethanolic methylamine (30 $cm^3$ w/v) and allowed to stand at ambient temperature for 5 days. From I.R. absorption spectrometry it appeared that the C=O ester absorption peak at 1735 $cm^{-1}$ had disappeared and the amide C=O absorption peak at 1670 $cm^{-1}$ had developed. The solution was evaporated to dryness followed by the addition of acetone (30 $cm^3$) which caused precipitation of $CH_3NH_3Cl$. The mother liquor was collected and evaporated to dryness. The addition of dry ether caused the precipitation of a further small amount of insoluble material. Evaporation of the ether left tiny crystals which were recrystallized from benzene/petroleum ether 60/80 to give 0.8 g (36%) of the desired product, m.p. 99—101°C.

Analysis:

| | | | |
|---|---|---|---|
| Calculated for $C_7H_{12}N_2O$: | C 59.5; | H 6.6; | N 20.0% |
| Found: | C 59.4; | H 8.7; | N 19.7% |

The specific rotation $[\alpha]_D^{23.5} = -164.4°$.

The starting material cis-L-2-ethoxycarbonyl-3,4-methano-pyrrolidine was obtained by mixing cis-L-2-carboxy-3,4-methano-pyrrolidine with dry ethanol and saturating the mixture with dry HCl-gas. The mixture was gently refluxed. After cooling, the solution was evaporated to almost dryness. More ethanol was added and $NH_3$-gas bubbled into the solution until no further precipitate occurred. The solution was filtered and evaporated to dryness leaving a semi-solid residue. Acetone was added which caused a further precipitate of $NH_4Cl$ and the filtered solution was evaporated to dryness. The product was extracted with diethyl ether and finally evaporated. The specific rotation of the pure ester $[\alpha]_D^{22} = -96°$.

Analysis:

| | | | |
|---|---|---|---|
| Calculated for $C_8H_{13}NO_2$: | C 61.9; | H 8.4; | N 9.0% |
| Found: | C 61.8: | H 8.7; | N 8.8% |

Example 2

Preparation of the hydrazide of cis-L-3,4-methano-pyrrolidine

Cis-L-2-ethoxycarbonyl-3,4-methano-pyrrolidine (1.3 g) in dry ethanol (20 $cm^3$) was treated with hydrazine (2 g) and refluxed for 5 hours. From I.R. absorption spectrometry it appeared that the C=O ester absorption peak at 1740 $cm^{-1}$ had disappeared and the amide C=O absorption peak at 1660 $cm^{-1}$ had developed. The solution was evaporated to dryness and the last traces of reagent and solvent were removed at reduced pressure (1 mm at 30°C). 1.1 g (93%) of the desired product was obtained as a viscous oil. The specific rotation $[\alpha]_D^{22} = -214°$.

Analysis:

| | | | |
|---|---|---|---|
| Calculated for $C_6H_{11}N_3O$: | C 51.0: | H 7.9; | N 29.8% |
| Found: | C 49.4; | H 7.9; | N 29.6% |

Example 3

Preparation of cis-L-N-formyl-2-ethoxycarbonyl-3,4-methano-pyrrolidine

Cis-L-2-ethoxycarbonyl-3,4-methano-pyrrolidine (1 g) was treated with the mixed anhydride of formic acid and acetic acid (1 g) in benzene (5 $cm^3$) under stirring. An exothermic reaction occurred. The solution was evaporated to dryness and finally treated in vacuo over KOH pellets to remove traces of acetic acid and mixed anhydride. 1.3 g of a pale brown viscous oil was obtained (100%). The specific rotation $[\alpha]_D^{19.5} = -142°$.

Analysis:

| | | | |
|---|---|---|---|
| Calculated for $C_{10}H_{16}N_2O_3$: | C 56.6; | H 7.6; | N 13.2% |
| Found: | C 55.8; | H 7.8; | N 13.0% |

Example 4

Preparation of cis-L-N-methylcarbamoyl-2-ethoxy carbonyl-3,4-methano-pyrrolidine

Cis-L-2-ethoxycarbonyl-3,4-methano-pyrrolidine (1 g) in dry benzene (5 $cm^3$) was treated with methyl isocyanate (1 g) and a trace of triethylamine under stirring. An exothermic reaction occurred. After stirring for 1 hour the mixture was evaporated to dryness followed by the addition of diethylether (20 $cm^3$). The product dissolved apart from 0.1 g insoluble material. The solution was filtered and evaporated to dryness leaving a viscous colourless oil of high purity (ester C=O 1750 $cm^{-1}$; urea C=O 1635 $cm^{-1}$). The yield was 1.4 g (100%). The specific rotation $[\alpha]_D^{19.2} = -113°$.

Analysis:

| | | | |
|---|---|---|---|
| Calculated for $C_{10}H_{16}N_2O_3$: | C 56.6; | H 7.6; | N 13.2% |
| Found: | C 55.8; | H 7.8; | N 13.0% |

Example 5

Preparation of cis-L-2-carboxy-3,4-methano-pyrrolidine hydrogen chloride acid salt hydrate and its methyl ester

(a) 0.5 g of natural cis-L-2-carboxy-3,4-methano-pyrrolidine was dissolved in water (5 $cm^3$) and treated with concentrated hydrochloric acid (5 $cm^3$). The solution was evaporated to dryness leaving a buff-coloured solid. The product was recrystallized from benzene/ethanol to give a white solid, m.p. 166—168°C.

Analysis:

| | | | |
|---|---|---|---|
| Calculated for $C_6H_{12}ClNO_3$: | C 39.7; | H 6.6; | N 7.7% |
| Found: | C 40.0; | H 6.9; | N 7.6% |

(b) By reacting cis-L-2-carboxy-3,4-methano-pyrrolidine HCl with methanol saturated with hydrogen chloride the corresponding cis-L-2-methoxycarbonyl-3,4-methano-pyrrolidine HCl was obtained, m.p. 123—125°C.

Analysis:

| | | | |
|---|---|---|---|
| Calculated for $C_7H_{12}ClNO_2$: | C 47.4; | H 6.8; | N 7.9% |
| Found: | C 46.5; | H 6.8; | N 7.7% |

Example 6

Preparation of cis-L-2-heptoxycarbonyl-3,4-methano-pyrrolidine hydrogen chloride acid salt

Cis-L-2-carboxy-3,4-methano-pyrrolidine (1.5 g) was suspended in pure n-heptanol and saturated with dry HCl gas. By continuous stirring a clear solution was slowly obtained. Removal of the excess n-heptanol under reduced pressure gave a viscous oil. 4.7 g of the desired product were obtained (9.7%). The specific rotation $[\alpha]_D^{20} = -38°$.

Analysis:

| | | | |
|---|---|---|---|
| Calculated for $C_{13}H_{24}ClNO_2$: | C 59.7; | H 9.2; | N 5.3% |
| Found: | C 58.9; | H 9.2; | N 5.2% |

Example 7

Preparation of trans-DL-N-tert.butyloxycarbonyl-2-carboxy-3,4-methanopyrrolidine

To a mixture of 8.2 g trans-DL-2-carboxy-3,4-methanopyrrolidine in 50% 1,4-dioxane/water (60 $cm^3$) and 13.5 $cm^3$ triethylamine 17.4 g $\alpha$-cyanobenzylidene-imino-(tert. butyl)-carbonate (BOC—ON) were added. The mixture was stirred for 2 hours during which period it became homogeneous.

Water (90 $cm^3$) and ethylacetate (120 $cm^3$) were added to the solution. After stirring the aqueous layer was separated. Subsequently, the organic layer was extracted with ethylacetate (120 $cm^3$), the aqueous layer was separated and the organic layer acidified with 5% citric acid solution. After 4 further extractions with ethylacetate (4 x 100 $cm^3$) the extracts were dried over $MgSO_4$ and evaporated to dryness, leaving a viscous oil of high purity which quickly solidified. The yield was 13.8 g (98%), the melting point 165°C (dec.). I.R. spect. C=O 1745 $cm^{-1}$ and 1640 $cm^{-1}$.

Analysis:

| | | | |
|---|---|---|---|
| Calculated for $C_{11}H_{17}NO_4$: | C 58.13; | H 7.54; | N 6.16% |
| Found: | C 57.8; | H 7.6; | N 6.2% |

## Example 8

Preparation of the trans-DL-N-tert. butyloxycarbonyl-2-dimethylcarbamoyl-3,4-methano-pyrrolidine

A mixture of trans-DL-N-tert. butyloxycarbonyl-2-carboxy-3,4-methano-pyrrolidine (2.3 g) and dry tetrahydrofuran (15 $cm^3$) was treated portionwise over 5 min. with carbodi-imidazole (1.74 g) with magnetic stirring. Effervescence was observed. The mixture was stirred for 1 hour, and treated with dimethylamine (0.5 g) in tetrahydrofuran (10 $cm^3$) in one portion. The orange-coloured solution was stirred overnight and evaporated to dryness. To the residue 35 $cm^3$ of 1N HCl were added and subsequently extraction with $CH_2Cl_2$ was carried out, followed by drying over $MgSO_4$ and evaporation to dryness. The oil thus obtained was redissolved in dry ether (100 $cm^3$), treated with animal charcoal and filtered. Evaporation left 2.0 g of a pale orange oil; yield 78%.

Analysis:

| | | | |
|---|---|---|---|
| Calculated for $C_{13}H_{22}N_2O_3$: | C 61.4; | H 8.7; | N 11.0% |
| Found: | C 61.3; | H 8.8; | N 10.5% |

I.R. spect. C=O at 1750 $cm^{-1}$ and 1050 $cm^{-1}$.

## Example 9

Preparation of trans-DL-N-tert. butyloxycarbonyl-2-ethylmercaptocarbonyl-3,4-methano-pyrrolidine

A mixture of trans-DL-N-tert. butyloxycarbonyl-2-carboxy-3,4-methano-pyrrolidine (4.3 g) and dry tetrahydrofuran (20 $cm^3$, dried over mol. sieves) was treated portionwise over 5 minutes with carbodi-imidazole (3.3 g). After 1 hour stirring when initial efferverscence had ceased, ethyl mercaptan (1.5 $cm^3$) was added. The orange-coloured solution was stirred for 15 hours at ambient temperature. After evaporation to dryness ice and 1N HCl (35 $cm^3$) were added and the mixture was stirred and extracted with $CH_2Cl_2$. Upon drying over $MgSO_4$ and evaporation an orange oil was left which was purified chromatographically using neutral $Al_2O_3$ and $CH_2Cl_2$ as eluent. The first fraction to emerge was collected (3.5 g); yield 73%.

Analysis:

| | | | |
|---|---|---|---|
| Calculated for $C_{13}H_{21}NSO_3$: | C 57.56; | H 7.7; | N 5.16% |
| Found: | C 57.6; | H 8.0; | N 5.1% |

I.R. spect. C=O at 1700 $cm^{-1}$ (strong)

## Example 10

Preparation of the hydrazide of trans-DL-2-carboxy-3,4-methano-pyrrolidine

A solution of ethyl ester of trans-DL-2-carboxy-3,4-methano-pyrrolidine (2.5 g) and hydrazine (95%, 2.5 g) in ethanol (50 $cm^3$) was allowed to stand at ambient temperatures for 1.5 days. Small needles were formed. The solution was evaporated to dryness leaving a white solid as residue which was re-crystallized from isopropanol. The yield was 1.5 g (68%), m.p. 130—1°C.

Analysis:

| | | | |
|---|---|---|---|
| Calculated for $C_6H_{11}N_3O$: | C 51.06; | H 7.8; | N 29.78% |
| Found: | C 51.2; | H 8.1; | N 30.1% |

## Example 11

Preparation of the methylamide of trans-DL-2-carboxy-3,4-methano-pyrrolidine

2.3 g of ethyl ester of trans-DL-2-carboxy-3,4-methanopyrrolidine were dissolved in ethanol (50 $cm^3$) and treated with an excess of 30% w/v of ethanolic methylamine (20 $cm^3$). The solution was allowed to stand for 2 days and was then refluxed for 2 hours and evaporated to dryness. A viscous oil remained (2 g); yield 95%.

Analysis:

| | | | |
|---|---|---|---|
| Calculated for $C_7H_{12}N_2O$: | C 60.0; | H 8.6; | N 20.0% |
| Found: | C 58.8; | H 8.3; | N 19.9% |

I.R. spect. C=O (broad) 1050 $cm^{-1}$
N—H (broad) 3320 $cm^{-1}$

Example 12

Preparation of cis-L-N-methyl-2-methoxycarbonyl-3,4-methano-pyrrolidine

To a cooled stirred solution of the methyl ester of cis-L-2-carboxy-3,4-methano-pyrrolidine (2.8 g) in dry diethyl ether (40 cm³) was added dropwise to a solution of $CH_2SO_3F$ ("magic methyl") (2.5 g) in dry diethyl ether (40 cm³). An instant reaction occurred with precipitation of a white salt. The mixture was stirred for a further 0.5 hour and then poured into a stirred cold saturated solution of NaCl and $NaHCO_3$ (100 cm³). After extraction (4x) with ether (4 × 100 cm³) the extracts were dried over $MgSO_4$ and evaporated to dryness, leaving a pale-yellow oil (2.5 g).

Analysis:

| | | | |
|---|---|---|---|
| Calculated for $C_8H_{13}NO_2$: | C 61.9; | H 8.4; | N 9.0% |
| Found: | C 62.2; | H 8.5; | N 8.9% |

The specification rotation $Na_D[\alpha]^{25}_{589} = -39.5°$.
I.R. spect. C=O 1750 $cm^{-1}$.
NMR spect. splitting of $H_a$ and $H_b$ was observed.

Example 13

Preparation of trans-DL-N-methyl-2-methoxycarbonyl-3,4-methano-pyrrolidine and hydrolysis thereof

To a cooled solution of the methyl ester of trans-DL-2-carboxy-3,4-methano-pyrrolidine (2.8 g) in dry diethyl ether (40 cm³) was added dropwise a solution of $CH_3SO_3F$ ("magic methyl") (2.5 g) in dry diethyl ether (10 cm³). An instant reaction occurred with separation of a heavy oil. The mixture was stirred for a further 1 hour and then poured into a stirred, cold, saturated solution of $NaHCO_3$/NaCl (100 cm³). After extraction with ether (4 × 100 cm³), the extracts were dried over $MgSO_4$. Evaporation of the filtered solution yielded the ester as an oil (1.5 g) of high purity.

The ester was hydrolysed by refluxing in 6N HCl (50 cm³) for 4 hours. The free amino acid could be isolated as a white solid using ion-exchange chromatography. The yield was 0.75 g, m.p. 200—1 °C (dec.).

Analysis:

| | | | |
|---|---|---|---|
| Calculated for $C_7H_{11}NO_2$: | C 59.55; | H 7.85; | N 9.9% |
| Found: | C 58.8; | H 7.9; | N 9.5% |

Example 14

Preparation of cis-L-N-methyl-2-carboxy-3,4-methano-pyrrolidine

The methyl ester of cis-L-N-methyl-2-carboxy-3,4-methano-pyrrolidine (1 g) was refluxed in 6N HCl (30 cm³) for 3 hours and then evaporated to dryness. The resulting crude amino acid-HCl was freed from its salt using DOWEX X 8—50 (Trademark) acid resin, followed by elution with 2N $NH_3$-solution. Evaporation of the solution under reduced pressure at about 50°C yielded an off-white crystalline solid (0.5 g), m.p. 218—20°C (dec.).
The specification rotation $Na_D[\alpha]^{25}_{589} = -37°C$.

Analysis:

| | | | |
|---|---|---|---|
| Calculated for $C_7H_{11}NO_2$: | C 59.55; | H 7.85; | N 9.7% |
| Found: | C 56.6; | H 8.2; | N 9.2% |

Calculated for the compound with 0.5 mol. $H_2O$ C 56.0; H 8.0; N 9.3%.

Example 15

Preparation of trans-DL-2-hydroxymethyl-3,4-methano-pyrrolidine

To a stirred slurry of $LiAlH_4$ ("lithal") (7.5 g) in dry diethyl ether (150 cm³) under $N_2$ was added dropwise a solution of the ethyl ester of trans-DL-2-carboxy-3,4-methano-pyrrolidine (20 g) in dry diethyl ether (50 cm³) at such a rate as to cause gentle reflux. After the addition period (15 min.) the reaction mixture was stirred and refluxed for 26 hours.

The mixture was cooled with ice-water and successively water (7.5 cm³), 15% NaOH (7.5 cm³) and water (22.5 cm³) were added. The mixture was stirred for 1 hour and the ether was decanted from the solid, which was washed with ether several times. Evaporation of the combined ether solutions yielded an almost colourless oil (11.5 g, 72%), which, after attempted distillation, solidified, m.p. 83—5°C.

Analysis:

| | | | |
|---|---|---|---|
| Calculated for $C_6H_{11}NO$: | C 63.7; | H 9.7; | N 12.4% |
| Found: | C 64.2; | H 10.1; | N 12.3% |

I.R. spect. no C=O.

Example 16

Preparation of cis-L-2-hydroxymethyl-3,4-methano-pyrrolidine

An amount of 4 g of cis-L-2-methoxycarbonyl-3,4-methano-pyrrolidine in dry diethyl ether (50 $cm^3$) was added to a stirred slurry of $LiAlH_4$ (2.0 g) of ether (800 $cm^3$) in such a way as to maintain a gentle reflux. The reduction was performed under a $N_2$ atmosphere.

After refluxing for 10 hours, the mixture was cooled and excess $LiAlH_4$ was decomposed by successively adding water (2 $cm^3$), 15% NaOH-solution (2 $cm^3$) and water (6 $m^3$). The mixture was filtered through "Super cell", dried over $MgSO_4$ and evaporated to dryness. The residue was an off-white solid (3.0 g), m.p. 72—4°C. The yield was 94%.

Analysis:

| | | | |
|---|---|---|---|
| Calculated for $C_6H_{11}NO$: | C 63.7; | H 9.7; | N 12.4% |
| Found: | C 63.5; | H 9.9; | N 11.9% |

Example 17

Preparation of the hydrochloric acid salt of trans-DL-2-n-heptyloxycarbonyl-3,4-methano-pyrrolidine

A mixture of trans-DL-2-carboxy-3,4-methano-pyrrolidine (1.5 g) and n-heptanol (20 $cm^3$) was saturated with dry HCl gas and stirred magnetically at 30°C for 6 hours. A clear solution resulted from which dissolved HCl-gas was removed by reducing the pressure. The excess n-heptanol was distilled off at 130°C/0.5 mm. The residue was a viscous oil.

Analysis:

| | | | |
|---|---|---|---|
| Calculated for $C_{13}H_{24}ClNO_2$: | C 59.65; | H 9.2; | N 5.35% |
| Found: | C 59.7; | H 9.4; | N 5.4% |

I.R. spect. C=O 1740 $cm^{-1}$.

Example 18

Demonstration of sterilizing activity

Tests were carried out with some pyrrolidine derivatives on spring wheat plants, variety "Sappo". The sterilants were applied to the plants at 3 stages of plant development, namely:

stage 7—second node visible
stage 8—last leaf just visible
stage 9—ligule of last leaf just visible.

Groups of plants (20 pots, 3 plants per pot) at the development stages 7—9 were sprayed with the respective sterilants as 300 ppm solutions in water with added wetter [TRITON X—155 (Trademark) concentration 0.1%]. Control tests with plants sprayed with water and wetter only were also carried out. At ear emergence a transparent bag was placed over the ears in order to prevent cross-pollination from untreated ears. The wheat plants were allowed to set seed and the average number of wheat grains per ear were counted, the results of these counts being shown in the Table below:

| Compound of Example | Average number of grains /ear at | | |
|---|---|---|---|
| | Stage 7 | Stage 8 | Stage 9 |
| untreated | 31.0 | 35.1 | 31.9 |
| 1 | 28.2 | 28.1 | 31.9 |
| 2 | 16.4 | 26.3 | 31.9 |
| 4 | 31.0 | 28.1 | 31.9 |
| 6 | 30.7 | 33.3 | 25.5 |

From the Table it will be seen that the compounds applied were most active at Stage 8.

**Claims**

1. 3,4-Methano-pyrrolidine derivatives of the general formula:

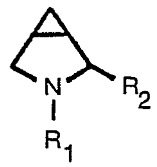

(I)

or the hydrogen halide acid addition salts thereof, wherein $R_1$ represents a hydrogen atom, an alkyl group, or a group of formula

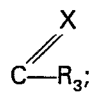

$R_2$ represents $CH_2OH$, a group of formula

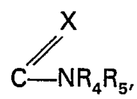

a group of formula

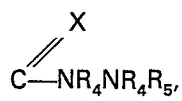

or a group of formula

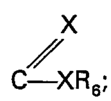

$R_3$ represents a hydrogen atom, an alkyl, alkoxy or alkylthio group, or a group of formula $—NR_4R_5$; each $R_4$ and $R_5$ independently represents a hydrogen atom or an alkyl group; $R_6$ represents a hydrogen atom, an alkyl group, a metal ion of (sub)group I or II of the Periodic Table, an ammonium ion or an alkyl-substituted ammonium ion; and each X independently represents oxygen or sulphur; with the proviso that if $R_1$ represents a hydrogen atom, $R_2$ does not represent a group of formula $CO_2R_6$.

2. Compounds as claimed in claim 1, in which $R_1$ represents a hydrogen atom, an alkyl group having up to 6 carbon atoms, or a group of formula

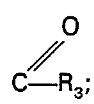

$R_2$ represents a group $CH_2OH$,

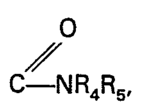 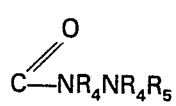 or 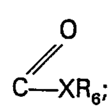

$R_3$ represents a hydrogen atom, an alkyl or alkoxy group having up to 6 carbon atoms, or a group $NR_4R_5$; and each of $R_4$ and $R_5$ independently represent a hydrogen atom or an alkyl group having up to 6 carbon atoms.

3. Compounds as claimed in claim 2, in which $R_1$ represents a methyl group or a hydrogen atom.

4. Hydrogen halide acid addition salts of compounds of the general formula I given in claim 1, in which $R_1$ represents a hydrogen atom and $R_2$ represents a group of formula $CO_2R_6$ where $R_6$ represents a hydrogen atom or an alkyl group.

5. A plant growth regulating and/or herbicidal composition comprising a carrier and/or surface

active agent and as active ingredient at least one compound as claimed in any one of claims 1 to 4.

6. A method for sterilizing male anthers in plants which comprises applying thereto an effective amount of at least one compound as claimed in any one of claims 1 to 4 or a composition as claimed in claim 5.

7. A method of controlling or eradicating weeds which comprises applying at a locus a herbicidally active amount of at least one compound as claimed in any one of claims 1 to 4 or a composition as claied in claim 5.

**Revendications**

1. Des dérivés de 3,4-méthanopyrrolidine de la formule générale:

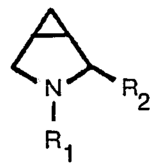

(I)

ou leurs sels d'addition d'acides halogènhydriques, où $R_1$ représente un atome d'hydrogène, un groupe alcoyle ou un groupe de formule

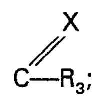

$R_2$ représente $CH_2OH$, un groupe de formule

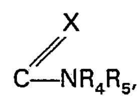

un groupe de formule

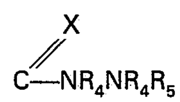

ou un groupe de formule

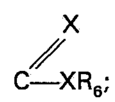

$R_3$ représente un atome d'hydrogène, un groupe alcoyle, alcoxy ou alcoylthio ou un groupe de formule $—NR_4R_5$; chaque $R_4$ et $R_5$ indépendamment représente un atome d'hydrogène ou un groupe alcoyle; $R_6$ représente un atome d'hydrogène, un groupe alcoyle, un ion de métal du groupe ou sous-groupe I ou II du tableau périodique, un ion d'ammonium ou un ion d'ammonium alcoylé; et chaque X indépendamment représente de l'oxygène ou du soufre; avec la condition que si $R_1$ représente un atome d'hydrogène, $R_2$ ne représente pas un groupe de formule $CO_2R_6$.

2. Des composés selon la revendication 1, dans lesquels $R_1$ représente un atome d'hydrogène, un groupe alcoyle ayant jusqu'à 6 atomes de carbone ou un groupe de formule

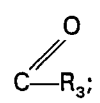

$R_2$ représente un groupe $CH_2OH$,

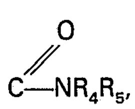 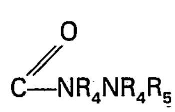 ou 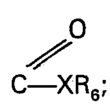

$R_3$ représente un atome d'hydrogène, un groupe alcoyle ou un groupe alcoxy ayant jusqu'à 6 atomes de carbone ou un groupe $NR_4R_5$; et chacun des $R_4$ et $R_5$ indépendamment représente un atome d'hydrogène ou un groupe alcoyle ayant jusqu'à 6 atomes de carbone.

3. Des composés selon la revendication 2, dans lesquels $R_1$ représente un groupe méthyle ou un atome d'hydrogène.

4. Des sels d'addition d'acide halogénhydrique de composés de la formule générale I indiquée dans la revendication 1, dans lesquels $R_1$ représente un atome d'hydrogène et $R_2$ représente un groupe de formule $CO_2R_6$ dans laquelle $R_6$ représente un atome d'hydrogène ou un groupe alcoyle.

5. Une composition pour régulation de la croissance des plantes et/ou herbicide comprenant un véhicule et/ou un agent tensio-actif et comme ingrédient actif au moins un composé selon l'une quelconque des revendications 1 à 4.

6. Un procédé pour stériliser les anthères mâles dans des plantes, qui comprend l'application aux plantes d'une quantité efficace d'au moins un composé selon l'une quelconque des revendications 1 à 4 ou d'une composition selon la revendication 5.

7. Un procédé pour lutter contre les mauvaises herbes ou pour les détruire, qui comprend l'application en un lieu d'une quantité active du point de vue herbicide d'au moins un composé selon l'une quelconque des revendications 1 à 4 ou d'une composition selon la revendication 5.

**Patentansprüche**

1. 3,4-Methanopyrrolidinderivate der allgemeinen Formel

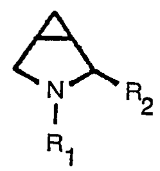 (I)

oder deren Halogenwasserstoff-Additionssalze, worin:
$R_1$ ein Wasserstoffatom, eine Alkylgruppe oder ein Gruppe der Formel

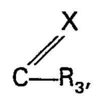

$R_2$ eine Gruppe der Formel $—CH_2OH$,

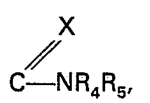 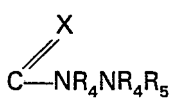 oder 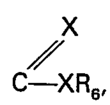

$R_3$ ein Wasserstoffatom, eine Alkyl-, Alkoxy- Alkylthiogruppe oder eine Gruppe der Formel $—NR_4R_5$ bedeuten, wobei
$R_4$ und $R_5$ unabhängig voneinander ein Wasserstoffatom oder eine Alkylgruppe und
$R_6$ ein Wasserstoffatom, eine Alkylgruppe, ein Ion eines Metalls der I. oder II. (Unter)Gruppe des Periodensystems oder ein gegebenenfalls alkyl-substituiertes Ammoniumion ist und
X ein Sauerstoff- oder Schwefelatom sein kann mit der Maßgabe, daß—wenn $R_1$ H ist—$R_2$ nicht $CO_2R_6$ sein kann.

2. Verbindungen nach Anspruch 1, worin $R_1$ ein Wasserstoffatom, eine Alkylgruppe mit bis zu 6 C-Atomen oder ein Gruppe der Formel

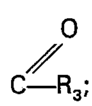

$R_2$—$CH_2OH$ oder eine Gruppe der Formel

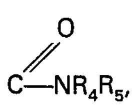 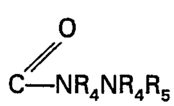 oder 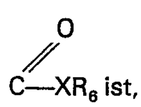 ist, wobei

$R_3$ ein Wasserstoffatom, eine Alkyl- oder Alkoxygruppe mit bis zu 6 C-Atomen oder die Gruppe $—NR_4R_5$ bedeutet und
$R_4$ und $R_5$ ein Wasserstoffatom oder eine Alkylgruppe bis zu 6 C-Atomen sein kann.

3. Verbindungen nach Anspruch 2, worin $R_1$ eine Methylgruppe oder ein Wasserstoffatom ist.

4. Halogenwasserstoff-Additionssalze der Verbindungen der allgemeinen Formel

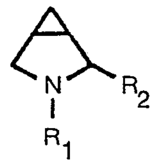

(I)

worin

$R_1$ ein Wasserstoffatom und

$R_2$ eine Groupe der Formel $—CO_2R_6$ ist, worin

$R_6$ ein Wasserstoffatom oder eine Alkylgruppe sein kann.

5. Mittel zur Regelung des Pflanzenwachstums und/oder Mittel mit Herbizidwirkung enthaltend einen Träger und/oder ein oberflächenaktives Mittel und als Wirkstoff mindestens eine der in Anspruch 1 bis 4 charakterisierten Verbindungen.

6. Verfahren zum Sterilisieren von männlichen Staubbeuteln in Pflanzen durch Aufbringen von mindestens einer der in Anspruch 1 bis 4 charakterisierten Verbindungen oder eines in Anspruch 5 charakterisierten Mittels.

7. Verfahren zur Kontrolle oder Ausrottung von Unkräutern durch Aufbringen von mindestens einer der in Anspruch 1 bis 4 charakterisierten Verbindungen bzw. eines in Anspruch 5 charakterisierten Mittels in einer eine Herbizidwirkung entfaltenden Menge auf den Standort.